(19) Europäisches Patentamt
European Patent Office
Office européen des brevets

(11) **EP 2 184 977 B1**

(12) **FASCICULE DE BREVET EUROPEEN**

(45) Date de publication et mention de la délivrance du brevet:
**12.07.2017 Bulletin 2017/28**

(51) Int Cl.:
**A01N 1/02** *(2006.01)*

(21) Numéro de dépôt: **08840369.6**

(22) Date de dépôt: **25.07.2008**

(86) Numéro de dépôt international:
**PCT/FR2008/001116**

(87) Numéro de publication internationale:
**WO 2009/050343 (23.04.2009 Gazette 2009/17)**

(54) **UTILISATION D'UNE GLOBINE, D'UN PROTOMERE DE GLOBINE OU D'UNE HEMOGLOBINE EXTRACELLULAIRE POUR LA PRESERVATION D'ORGANES, DE TISSUS, OU DE CELLULES D'ORGANES OU DE TISSUS**

VERWENDUNG VON GLOBIN, EINEM GLOBINPROTOMER ODER EINEM EXTRAZELLULÄREN HÄMOGLOBIN ZUR KONSERVIERUNG VON ORGANEN, GEWEBEN UND GEWEBEZELLEN

USE OF A GLOBIN, A GLOBIN PROTOMER OR AN EXTRACELLULAR HEMOGLOBIN FOR THE PRESERVATION OF ORGANS, TISSUES, ORGAN AND TISSUE CELLS

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **09.08.2007 FR 0705804**

(43) Date de publication de la demande:
**19.05.2010 Bulletin 2010/20**

(73) Titulaires:
• **Hemarina SA
29679 Morlaix Cedex (FR)**
• **Centre National de la Recherche Scientifique
75794 Paris Cedex 16 (FR)**
• **Université Pierre et Marie Curie (Paris 6)
75005 Paris (FR)**

(72) Inventeurs:
• **ZAL, Franck
29600 Ploujean-Morlaix (FR)**
• **ROUSSELOT, Morgane
29250 Saint Pol de Leon (FR)**
• **DUTHEIL, Delphine
86800 Saint Julien l'Ars (FR)**

(74) Mandataire: **Lavoix
2, place d'Estienne d'Orves
75441 Paris Cedex 09 (FR)**

(56) Documents cités:
**WO-A-01/92320          WO-A-2007/085596**

**DE-A1- 10 220 990          FR-A- 2 860 796**

• **ROUSSELOT, MORGANE ET AL: "Arenicola marina extracellular hemoglobin: a new promising blood substitute" BIOTECHNOLOGY JOURNAL, vol. 1, no. 3, 2006, pages 333-345, XP002480915**
• **PIONETTI J-M ET AL: "Molecular architecture of annelid erythrocruorins . Extracullular hemoglobin of arenicola marina (Polychaeta)" EUROPEAN JOURNAL OF BIOCHEMISTRY, BERLIN, vol. 105, no. 1, 1 janvier 1980 (1980-01-01), pages 131-138, XP009026718 ISSN: 0014-2956**
• **Lionel Badet ET AL: "L'utilisation des liquides de conservation en transplantation rénale", Progrès en Urologie, 1 January 2006 (2006-01-01), pages 25-31, XP055335201, Retrieved from the Internet: URL:https://www.google.fr/url?sa=t&rct=j&q =&esrc=s&source=web&cd=1&ved=0ahUKEwju 18Ga 977RAhXCOxoKHVTYALcQFggaMAA&url=http:/ /www .urofrance.org/sites/default/files/fileadm in/documents/data/PU/2006/PU-2006-00160025 /TEXF-PU-2006-00160025.PDF&usg=AFQjCNHB 0sj OaEY6k25E6VjoNm5Pa_buWA&bvm=bv.144224 172,d .d2s [retrieved on 2017-01-13]**

Remarques:
    Le dossier contient des informations techniques présentées postérieurement au dépôt de la demande et ne figurant pas dans le présent fascicule.

Remarques:
    Le dossier contient des informations techniques présentées postérieurement au dépôt de la demande et ne figurant pas dans le présent fascicule.

**Description**

La présente invention décrit l'utilisation d'au moins une globine, et/ou d'au moins un protomère de globine et/ou d'au moins une hémoglobine native, naturellement extracellulaire, pour la préservation d'organes, de tissus, ou de cellules d'organes ou de tissus, ou de culture de cellules.

On a beaucoup étudié les Annélides pour leur hémoglobine extracellulaire (N.B. Terwilliger, Molecular Structure of the extracellular heme proteins., Vol. 13, C.P. Mangum (Ed), 193-229, Springer-Verlag, Berlin (1992); J.N. Lamy, B.N. Green, A. Toulmond, J.S. Wall, R.E. Weber and S.N. Vinogradov, Chem. Rev. 96 3113-3124 (1996)). Ces molécules d'hémoglobine extracellulaire sont présentes chez les trois classes d'Annélides: Polychètes, Oligochètes et Achètes et même chez les Vestimentifères devenue récemment la famille des siboniglidae incluse à la classe des Polychètes. Ce sont des biopolymères géants, constitués d'environ 200 chaînes polypeptidiques appartenant à 6 ou 8 types différents que l'on regroupe généralement en deux catégories. La première catégorie, comptant 144 à 192 éléments, regroupe les chaînes polypeptidiques dites "fonctionnelles" portant un site actif et capables de lier réversiblement l'oxygène ; ce sont des chaînes de type globine dont les masses sont comprises entre 15 et 18 kDa et qui sont très similaires aux chaînes de type $\alpha$ et $\beta$ de vertébrés. La deuxième catégorie, comptant 36 à 42 éléments, regroupe les chaînes polypeptidiques dites de "structure" possédant peu ou pas de site actif mais permettant l'assemblage des douzièmes.

Les premières images obtenues sur des hémoglobines extracellulaires d'Arénicole (J. Roche, M. Bessis and J.P. Thiery, Biochim. Biophys. Acta 41 182-184 (1960); J. Roche, M.T. Bessis and J.P. Thiery, C. R. Soc. Biol. 154 73-80 (1960)) ont révélé des éléments hexagonaux. Chaque molécule d'hémoglobine est constituée de deux hexagones superposés (O. Levin, J. Mol. Biol. 2. 95-101 (1963); J. Roche, Electron microscope studies on high molecular weight erythrocruorins (invertebrate hemoglobins) and chlorocruorins of annelids., D.A. Munday (Ed), 62-80, Pergamon Press, Oxford (1965)) que l'on a nommés bicouche hexagonale (hexagonal bilayer) et chaque hexagone est lui-même formé par l'assemblage de six éléments en forme de goutte d'eau (E.F.J. Van Bruggen and R.E. Weber, Biochim. Biophys. Acta 359 210-212 (1974); O.H. Kapp and A.V. Crewe, Biochim. Biophys. Acta 789 294-301 (1984)), nommés structure globulaire creuse (hollow globular structure) (F. De Haas, F. Zal, V. You, F.H. Lallier, A. Toulmond and J.N. Lamy, J. Mol. Biol. 264 111-120 (1996); F. De Haas, F. Zal, F.H. Lallier, A. Toulmond and J.N. Lamy, Proteins-structure fonction and genetics, 3 241-256 (1996); F. De Haas, N. Boisset, J.C. Taveau, O. Lambert, S.N. Vinogradov and J.N. Lamy, Biophys. J. 70 1973-1984 (1996)) ou "douzième". La molécule native est formée de douze de ces sous-unités (dodécamère), d'une masse moléculaire comprise entre 200 et 250 kDa qui constitue l'unité fonctionnelle de la molécule native. On s'est particulièrement intéressé à *Arenicola marina,* un Annélide Polychète de l'écosystème intertidal. La structure de son hémoglobine extracellulaire est d'ailleurs déjà connue (F. Zal, B. N. Green, F.H. Lallier, S.N. Vinogradov and A. Toulmond, Eur. J. Biochem. 243 85-92 (1997)). WO01/92320 et Rousselot et al (Biotechnology Journal, vol.1, no.3, 2006, pages 333-345) divulguent l'utilisation d'hémoglobine d'*Arenicola marina* comme substitut sanguin. DE10220990 décrit l'utilisation de myoglobines et d'hémoglobines dans la culture de cellules. WO2007/085596 divulgue l'utilisation d'hémoglobine d'*Arenicola marina* pour traiter les désordres liés au calcium. Badet al. (L'utilisation des liquides de conservation en transplantation rénale, Prog Urol, 2006, 16, 25-31) décrit des recommandations d'utilisation de liquides de conservation d'organe.

La transplantation d'organe consiste à remplacer un organe malade d'un patient par un organe sain, appelé greffon et provenant d'un donneur.

La distribution de l'oxygène à tous les organes et tissus dans le corps humain est assurée par l'hémoglobine présente dans le flux sanguin humain ou animal.

Après prélèvement sur le donneur, l'organe n'est plus approvisionné en oxygène et donc, lors du processus de transplantation d'organe, le maintien en vie, le plus longtemps possible, de l'organe hors du corps pendant le temps qui sépare le prélèvement sur le donneur, de l'implantation sur le patient, est indispensable à la réussite de la greffe et implique la mise au point de méthodes de conservation.

Dès l'explantation, le greffon est privé de son environnement physiologique et devient alors très sensible. En normothermie (37°C), l'interruption de la vascularisation d'un organe entraîne la nécrose rapide des cellules qui le constituent. La solution de préservation d'organe intervient afin de le protéger. L'un des grands principes de la préservation d'organe consiste à descendre rapidement sa température de 37°C à 4°C. En effet, la diminution de la température des tissus entraîne une diminution du métabolisme cellulaire, sans pour autant l'arrêter (Belzer F.O., Southard J.H. Principles of solid-organ preservation by cold storage. Transplantation 1988; 45(4): 673-676.). L'hypothermie et la composition de la solution de conservation permettent de lutter contre les effets délétères de la privation en oxygènes et nutriments induits par l'arrêt de la circulation sanguine et diffèrent la mort des cellules, responsable de la nécrose des tissus. La solution contribue ainsi, grâce à ses propriétés osmotiques et/ou anti-oxydantes, au maintien de la qualité et de l'intégrité du greffon (morphologie et biochimie). Surtout elle maintient sa viabilité *ex vivo* :

- Lors de la phase de prélèvement, le greffon va être perfusé par la solution de préservation afin de le rincer et le débarrasser du sang du donneur (« solution de rinçage »). L'organe est refroidi suite à la perfusion hypothermique réalisée *ex vivo* ou *in situ* selon qu'il s'agit d'un prélèvement sur donneur vivant ou cadavérique. Le lavage sert également à équilibrer l'organe avec les composants de la solution.

- La période, depuis le début de l'explantation chez le donneur jusqu'à la fin de l'implantation chez le receveur, est critique : c'est la durée d'ischémie totale. Cette étape est à l'origine des nombreux effets délétères observés. L'ischémie peut se définir comme une insuffisance d'apport sanguin tissulaire, avec perte de trois fonctions importantes du flux sanguin: apport de nutriments, oxygénation et élimination des déchets.

On distingue le temps d'ischémie chaude : période où l'organe n'est plus perfusé par le sang du donneur mais pas encore réfrigéré, et le temps d'ischémie froide : période après lavage et réfrigération de l'organe jusqu'à sa revascularisation chez le receveur. Après réchauffement de l'organe et avant perfusion par le sang du receveur, on peut observer une période d'ischémie chaude secondaire. La solution de préservation a un rôle réel de protection du greffon pendant le transport (« solution de stockage »).

L'hypothermie est l'élément essentiel de la conservation. Elle réduit le métabolisme tissulaire, c'est-à-dire qu'elle ralentit l'activité enzymatique catalytique nécessaire à la viabilité cellulaire. Le métabolisme serait diminué de 12 à 13 fois lorsque la température passe de 37°C à 0°C (Belzer et Southard, Principles of solid-organ preservation by cold storage.Transplantation 1988; 45(4): 673-676. 1988)).

Il est en effet nécessaire de réduire la demande et la consommation du greffon en oxygène et en énergie, car celui-ci se trouve en état d'ischémie. Cela sous entend que le tissu est privé d'oxygène. Donc la synthèse d'énergie sous forme d'ATP n'est plus assurée par la phosphorylation oxydative mais par la glycolyse anaérobie dont le rendement est très inférieur. Le tissu ischémique n'a presque plus de réserve énergétique et se détériore rapidement.

Il est donc important de réduire ses besoins grâce à l'hypothermie. La qualité de la conservation à froid (~4°C) conditionnera la réussite de la reperfusion après l'implantation. L'organe est simplement plongé dans la solution maintenue à basse température par de la glace pilée selon des conditions garanties d'asepsie : conservation statique à froid.

Le temps acceptable pour assurer la reprise de fonction ultérieure du greffon varie d'un organe à l'autre. Par exemple, il est d'environ 4-5 heures pour le coeur, 4-6 heures pour le poumon, 6 heures pour l'intestin, 10-16 heures pour le foie, 24-35 heures pour le rein et 12-18 heures pour le pancréas (Thèse de Melle Delphine FORNAS soutenue le 15 juin 2001 : Solution de préservation d'organe : descriptif, statut réglementaire et enregistrement en Europe ; Université Claude Bernard - Lyon I ; Faculté de Pharmacie ; Institut des Sciences Pharmaceutiques et Biologiques).

Par conséquent, la mise au point de méthodes de conservation a été particulièrement étudiée.

Ainsi, le brevet US 7,220,538 concerne une composition de préservation d'organes ou de cellules, à deux phases, et comprenant une première phase qui comprend un milieu de base nutritif et une deuxième phase qui comprend des nanoparticules contenant une solution ou suspension avec un composant capable de lier ou fournir de l'oxygène qui peut être de l'hémoglobine intracellulaire hémolysée et modifiée chimiquement. Cette solution est maintenue a une température non hypothermique d'environ 20 à 37°C.

Le brevet US 6,994,654 concerne une solution de préservation d'organe et tissus qui contient une solution à base d'électrolytes à haute teneur en potassium et un additif qui peut être du PEG-Hémoglobine pour un procédé de fourniture, préservation, transplantation et/ou de chirurgie non hémorragique impliquant un organe ou un tissu. Il est précisé que les molécules transportant l'oxygène, utilisées comme additif, le sont pour une perfusion « normothermique ». Lorsque la perfusion est hypothermique, il n'y a pas de présence de transporteur d'oxygène car aux températures hypothermiques, l'hémoglobine de vertébrés dont est issue la molécule décrite dans ce brevet n'a pas les mêmes proprieté fonctionnelles qu'à 37°C, notamment son affinité vis à vis de l'oxygène car l'affinité de l'hémoglobine pour l'oxygène dépend de la température.

La demande de brevet US 2006/0063142 concerne un appareil et un procédé de perfusion d'organe pour le suivi, le maintien et/ou la restauration des organes ainsi que la préservation des organes durant le stockage et/ou le transport.

Le procédé comprend la perfusion d'un organe avec un premier fluide médical à une première température (préférentiellement supérieure à 25°C) qui peut contenir un transporteur d'oxygène tel que les globules rouges ou de l'hémoglobine réticulée, après rinçage avec une solution qui peut être du VIASPAN™ ou d'autres solutions colloïdales contenant du dextran ou du HES (hydroxyethyl starch) ou d'autres composés équivalents, puis la perfusion de l'organe avec un deuxième fluide médical qui ne contient pas d'oxygène à une seconde température (préférentiellement comprise entre 4 et 10°C) inférieure à la première.

Le brevet US 6,642,045 concerne un système d'assistance métabolique comprenant un organe ou un tissu qui emploie une solution de perfusion qui peut comprendre un transporteur d'oxygène tel que l'hémoglobine, une hémoglobine stabilisée, des conjugués de polyoxyéthylène d'hémoglobine ou une hémoglobine recombinante. La température d'utilisation du système est comprise entre 25 et 37°C.

La demande WO 01/01774 concerne une composition pour la préservation d'organes pour la transplantation contenant une PEG-hémoglobine bovine, un ou plusieurs électrolytes essentiels, au moins une protéine soluble, au moins une formulation nutritionnelle et au moins un agent agissant sur le système cardiovasculaire.

Dans ces différents documents, l'hémoglobine utilisée est d'origine humaine ou de mammifères et est soit dans les globules rouges, soit dans des liposomes, ou alors elle est réticulée, ou pontée par du polyéthylène glycol (PEG-hémoglobine), pour éviter l'oxydation car il est bien connu que lorsque l'on isole une hémoglobine du globule rouge, celle-ci va s'oxyder du fait de l'absence de l'activité anti-oxydante des enzymes présentes dans le globule rouge. (Savitsky JP, Doczi J, Black j & Arnold JD (1978) A clinical safety trial of stroma-free hemoglobin. Clin Pharmacol Ther 23, 73-80), (Chan WL, Tang NL, Yim CC, Lai FM & Tam MS (2000); New features of renal lesion induced by stroma free haemoglobin, Toxicol Pathol 28, 635-642). L'utilisation de globules rouges nécessite également le contrôle de la pression osmotique. Au-delà de 289 mOsMoles (pression osmotique normale du globule rouge), le globule rouge va se trouver dans un milieu hyperosmotique et aura tendance à perdre l'eau qu'il contient. Ce processus physique pertube les échanges membranaires notamment les échanges sodium/chlore (Hendry EB (1961) Osmolarity of human serum and chemical solution of biological importance. Clin. Chem., 2, 156-164).

Egalement, l'utilisation d'une hémoglobine humaine ou de mammifère n'autorise que des températures normothermiques pour la préservation d'organe car en deçà, le fonctionnement du globule rouge est fortement perturbé (Jensen, FB, Wang T., Brahm, J., 2001, Acute and chronic influence of temperature on red blood cell anion exchange, 204, 39-45). Par ailleurs, l'utilisation d'une hémoglobine de vertébrés nécessite de connaître le typage sanguin du donneur et du receveur afin d'éviter une réaction immunologique (Goodnough,Clin Orthop RelatRes. 1998Dec;(357):89-100).

Cependant, l'amélioration de la survie du greffon pendant l'ischémie sur une période plus longue permettra une meilleure étude de l'immunologie et des conditions opératoires, favorisant ainsi la réussite de la greffe.

De même, l'amélioration de l'oxygénation du greffon et donc de sa qualité permettraune reprise de fonction plus rapide.

Par conséquent, l'un des objets de l'invention est de fournir des globines, et/ou des protomères de globine et/ou des hémoglobines extracellulaires en association avec un milieu de conservation d'organe permettant la constitution d'une composition de préservation d'organes, de tissus, ou de cellules d'organes ou de tissus, ou de culture de cellules , telle que définie dans la revendication 1.

Un autre objet de l'invention est de fournir une composition de préservation d'organes, de tissus, ou de cellules d'organes ou de tissus, telle que définie dans la revendication 1, ne nécessitant pas de typage sanguin.

Un autre aspect de l'invention est de fournir une composition de préservation d'organes, de tissus ou de cellules d'organes ou de tissus, telle que définie dans la revendication 1, permettant une utilisation prolongée dans le temps et pouvant fonctionner à une température hypothermique.

Il est également décrit une composition de culture de cellules pouvant fonctionner à une température hypothermique.

Un autre objet de l'invention est de fournir une composition comprenant au moins une globine et/ou au moins un protomère de globine et/ou au moins une hémoglobine extracellulaire dans un milieu de conservation d'organe, telle que définie à la revendication 1, permettant la préservation d'organes, de tissus ou de cellules d'organes ou de tissus ou la culture de cellules.

Un autre objet de l'invention est de fournir un procédé de conservation d'organes, de tissus ou de cellules d'organes ou de tissus, tel que défini à la revendication 8.

Il est également décrit un procédé de perfusion d'organe.

Il est également décrit un procédé de culture de cellules.

Par conséquent, l'invention décrit l'utilisation d'au moins une globine et/ou d'au moins un protomère de globine et/ou d'au moins une hémoglobine native naturellement extracellulaire d'animal invertébré choisi parmi le phylum des annélides et notamment au moins une globine et/ou au moins un protomère de globine et/ou au moins une hémoglobine extracellulaire appartenant à des vers marins tels que *Arenicola marina,* à une concentration, par rapport au volume final, comprise de 0,625 mg/ml à 100 mg/ml, préférentiellement de 0,625 mg/ml à 20 mg/ml, plus préférentiellement de 0,625 mg/ml à 5 mg/ml, en particulier 1,25 mg/ml, en association avec un milieu de conservation d'organe, ou un milieu de culture de cellules, pour la constitution d'une composition de préservation d'organes, de tissus, ou de cellules d'organes ou de tissus, ou de perfusion d'organe ou de tissus, ou de culture de cellules.

Par « protomère de globine », on entend le douzième ou le dodécamère de la globine.

Par «native», on entend une globine, ou un protomère de globine ou une hémoglobine originaire dudit animal invertébré.

Par « naturellement extracellulaire », on entend une globine ou un protomère de globine ou une hémoglobine qui est naturellement non contenue dans une cellule et peut donc circuler librement dans le système circulatoire sans modification chimique pour la stabiliser et la rendre fonctionnelle.

L'hémoglobine extracellulaire de *Arenicola marina* est un biopolymère géant d'une masse d'environ 3 à 4 millions de daltons et composé d'environ 200 chaînes polypeptidiques de deux types. Les trois quarts sont des chaînes de type globines capables de fixer réversiblement l'oxygène ($O_2$) et le quart restant sont des chaînes de structures (« linkers ») qui assurent le maintien de la structure quaternaire et seraient responsable de l'activité anti-oxydante de cette molécule. L'unité fonctionnelle de cette molécule est le docécamère qui a une masse comprise entre 200 et 250 kDa.

Par conséquent, l'hémoglobine extracellulaire, ou le protomère de globine ou la globine d'animal invertébré peut être constituée d'une seule chaîne polypeptidique jusqu'à environ plusieurs centaines de chaînes polypeptidiques, soit un poids moléculaire compris d'environ 15 000 Daltons à environ 8 Millions de Daltons.

L'utilisation d'au moins une globine et/ou d'au moins un protomère de globine et/ou d'au moins une hémoglobine native extracellulaire permet de mettre enjeu l'activité superoxide dismutase (SOD) intrinsèque (déterminée par la méthode de Flohé & Ötting ; Flohé L, Otting F. Methods Enzymol (1984), 105, 93-104) de ladite hémoglobine ou globine ou protomère de globine, procurant ainsi une activité anti-oxydante intrinsèque, et ne nécessitant, par conséquent, aucun anti-oxydant pour fonctionner, contrairement à l'utilisation d'une hémoglobine de mammifères pour laquelle les molécules anti-oxydantes sont contenues à l'intérieur du globule rouge et ne sont pas liées à l'hémoglobine. D'autre part, la globine ou le protomère de globine ou l'hémoglobine extracellulaire ne nécessitent pas de cofacteur pour fonctionner contrairement à l'hémoglobine de mammifère, notamment humaine.

Par animal invertébré, on entend un animal qui n'a pas de colonne vertébral tel que les méduses, les éponges, les insectes, les crustacés, les mollusques... ou un animal qui appartient à l'embranchement des annélides.

La concentration en hémoglobine dans le ver marin *Arenicola marina* est comprise entre 100 à 170 g/L de sang (Toulmond, A. (1975). Recherches sur la physiologie respiratoire de l'Annélide Polychète Arenicola marina (L.). Thèse de Doctorat d'Etat, Pierre-et-Marie-Curie (Paris VI), Paris) et chez l'homme, elle est d'environ 140 g/L de sang (Données OMS WHO/LEISH/96.40 Appendice5 Page 57). Par conséquent, les concentrations utilisées dans l'invention sont préférentiellement d'environ 100 fois inférieures aux concentrations physiologiques des invertébrés ou vertébrés.

L'utilisation d'au moins une globine et/ou d'au moins un protomère de globine et/ou d'au moins une hémoglobine native extracellulaire évite le contrôle de la pression osmotique nécessaire avec l'utilisation de globules rouges. La globine, le protomère de globine et l'hémoglobine extracellulaire ne possédant pas de typage sanguin permettent également d'éviter tout problème de réaction immunologique rencontré avec l'utilisation de globule rouge de mammifère telle que hémoglobine humaine ou de bovin contenue dans des hématies qui possèdent à leur surface différents types glycanes responsable du typage sanguin. L'hémoglobine extracellulaire de *A. marina* étant extracellulaire et non glycosilée, *A. marina* peut être considéré comme un donneur universel.

Les organes et tissus sont d'origine animale, notamment humaine, de mammifères, d'oiseaux, de reptiles, de poissons ou d'insectes.

Un tissu est un ensemble de cellules identiques ou tout au moins de même origine, participant à une fonction commune. Les tissus se groupent en organes.

L'expression « milieu de conservation » désigne tout milieu capable de protéger les organes et/ou les cellules des effets délétères de l'ischémie reperfusion en satisfaisant les besoins métaboliques minimums des organes et/ou cellules.

Les milieux de conservation sont des solutions aqueuses contenant des électrolytes tels que le potassium, le sodium, le magnésium, le calcium, le chlorure, le sulfate, contenant le cas échéant des imperméants tels que le mannitol, le raffinose, le saccharose, le glucose, le fructose, le lactobionate, ou du gluconate, et peuvent également contenir des colloïdes tels que de l'abumine, de l'hydroxyéthyl amidon, du polyéthylène glycol ou du dextran 40.

Les milieux de culture de cellules sont disponibles commercialement et très variés. Par exemple, des milieux disponibles chez Invitrogen, sans être limités à ceux-ci sont les milieux suivants: D-MEM, D-MEMIF-12, MEM, RPMI 1640, ou milieu 199... ou tout milieu analogue.

Des exemples de solutions, sans limiter l'invention à ceux-ci, sont donnés dans le tableau 1 de la thèse de Melle Delphine Fornas (Thèse de Melle Delphine FORNAS soutenue le 15 juin 2001 Solution de préservation d'organe descriptif, statut réglementaire et enregistrement en Europe ; Université Claude Bernard - Lyon I ; Faculté de Pharmacie; Institut des Sciences Pharmaceutiques et Biologiques). Par culture de cellules, il faut entendre tous type de cellules, notamment les cellules procaryotes et eucaryotes, telles que des micro-organismes libres (bactéries ou levures), des cellules "saines" prélevées fraîchement d'un organisme (biopsie, ...), qui constituent alors des "cultures primaires".

Il faut entendre également des cellules ayant une capacité de division non limitée (on parle d'"immortalité en culture"), par exemple les lignées de cellules cancéreuses, de cellules en voie de cancérisation, ou encore des cellules saines rendues "immortelles" artificiellement.

Selon un mode de réalisation préféré de l'invention, la globine, et/ou le protomère de globine, et/ou l'hémoglobine native défini ci-dessus est utilisé avec un milieu de conservation d'organe choisi préférentiellement parmi la solution de l'Université du Wisconsin (UW, Viaspan"'), IGL1®, Celsior®, SCOT Maco®, BMPS Belzer®, Custodiol® (HTK), Euro-Collins®, Soltran®, Perfadex®, Ringer lactate®, ou Plegisol®, ou toute solution analogue,

Tous ces milieux de conservation d'organe sont des produits commerciaux.

La culture de cellules définie ci- dessus peut correspondre à des cellules de vertébrés.

Par vertébré, on désigne un mammifère, un reptile, un amphibien, un oiseau ou un poisson.

Les cellules de vertébrés correspondent à tous type de cellules appartenant à un animal vertébré et peuvent être, par exemple, sans être limité à celles-ci, des cellules rénales, hépatiques, pancréatiques, cardiaques, pulmonaires, intestinales, de l'estomac, du colon ....

La culture de cellules définie ci-dessus peut correspondre à des cellules d'invertébrés.

Par invertébré on désigne un animal dépourvu de colonne vertébrale, par exemple les insectes, les mollusques, les annélides, les cnidaires, les spongiaires, ...

Les cellules d'invertébrés correspondent à tous type de cellules appartenant à un animal invertébré et peuvent être, par exemple, sans être limité à celles-ci, des cellules du tissu périvasale/hématopoïétique ou autres.

Selon un mode de réalisation préféré, la température des compositions de préservation d'organe ou de cellules d'organes, ou de culture de cellules définies ci-dessus, est comprise de 4°C à 37°C, préférentiellement de 4°C à 25°C, plus préférentiellement de 4°C à 15°C, en particulier 4°C.

Ainsi l'utilisation d'au moins une globine et/ou d'au moins un protomère de globine et/ou d'au moins une hémoglobine native extracellulaire permet de constituer une composition capable de travailler en condition hypothermique, élément essentiel de la conservation, car l'hypothermie réduit le métabolisme tissulaire, c'est-à-dire qu'elle ralentit l'activité enzymatique catalytique nécessaire à la viabilité cellulaire.

L'utilisation d'au moins une globine et/ou d'au moins un protomère de globine et/ou d'au moins une hémoglobine, native extracellulaire, permet également de cultiver des cellules d'invertébrés marins, ce qui actuellement n'est pas possible. En effet, des cultures primaires d'invertébrés marins sont réalisées sur certains types d'invertébrés (certaines sont maintenues plusieurs mois) mais aucune lignée de cellule n'a été établie. (Rinkevich B, Mar Biotechnol (NY). 2005 Sep-Oct;7(5):429-39).

Selon un autre mode de réalisation, la température de la composition de perfusion d'organe ou de culture de cellules est comprise de 4°C à 37°C, préférentiellement d'environ 15°C à environ 37°C, plus préférentiellement de 25°C à 37°C, en particulier 37°C.

Lors de la perfusion d'un organe, la température de perfusion doit être généralement normothermique, c'est-à-dire proche de la température physiologique. Par conséquent, l'utilisation d'une hémoglobine extracellulaire permet de constituer une composition capable de travailler également en condition normothermique.

Selon un autre aspect, l'invention concerne une composition telle que définie dans la revendication 1 comprenant au moins une globine et/ou au moins un protomère de globine et/ou au moins une hémoglobine, native extracellulaire, d'animal invertébré choisi parmi le phylum des annélides et notamment au moins une globine et/ou au moins un protomère et/ou au moins une hémoglobine extracellulaire appartenant à des vers marins tels que *Arenicola marina*, à une concentration, par rapport au volume final, comprise de 0,625 mg/ml à 100 mg/ml, préférentiellement de 0,625 mg/ml à 20 mg/ml, plus préférentiellement de 0,625 mg/ml à 5 mg/ml, en particulier 1,25 mg/ml; et un milieu de conservation d'organes.

Ladite composition permet de préserver des organes ou des tissus, ou des cellules d'organes ou de tissus, ou la perfusion d'organe ou de tissus.

Selon un mode de réalisation préféré, ledit milieu de conservation d'organe est choisi préférentiellement parmi la solution de l'Université du Wisconsin (UW, Viaspan"), IGL1®, Celsior®, SCOT Maco®, BMPS Belzer®, Custodiol® (HTK), Euro-Collins®, Soltran®, Perfadex®, Ringer lactate®, ou Plegisol®, ou toute solution analogue, mais n'est pas limité à ces seules solutions colloïdales.

Selon un autre aspect, il est décrit une composition comprenant au moins une globine et/ou au moins un protomère de globine et/ou au moins hémoglobine, native extracellulaire, d'animal invertébré choisi parmi le phylum des annélides et notamment au moins une globine et/ou au moins un protomère de globine et/ou au moins une hémoglobine extracellulaire appartenant à des vers marins tels que *Arenicola marina,* à une concentration, par rapport au volume final, comprise de 0,625 mg/ml à 100 mg/ml, préférentiellement de 0,625 mg/ml à 20 mg/ml, plus préférentiellement de 0,625 mg/ml à 5 mg/ml, en particulier 1,25 mg/ml; et un milieu de culture de cellules.

Ladite composition permet la culture de cellules de vertébrés ou d'invertébrés. Selon encore un autre aspect, l'invention concerne un procédé de conservation d'organe ou de tissu, ou de cellules d'organes ou de tissus comprenant une étape de conservation en statique ou en perfusion dynamique dudit organe dans une composition telle que définie dans la revendication 1.

Dans un mode de réalisation préféré, le procédé de conservation d'un organe ou de tissus, ou de cellules d'organes ou de tissus, défini ci-dessus, comprend les étapes suivantes:

- prélèvement dudit organe ou dudit tissu, ou desdites cellules d'organes ou de tissus;
- rinçage dudit organe ou dudit tissu, ou desdites cellules d'organes ou de tissus, à une température comprise de 4°C à 37°C, préférentiellement de 4°C à 25°C, plus préférentiellement de 4°C à 15°C, en particulier 4°C, avec une composition définie ci-dessus;
- conservation en statique ou en perfusion dynamique dudit organe ou dudit tissu, ou desdites cellules d'organes ou de tissus, à une température comprise de 4°C à 37°C, préférentiellement de 4°C à 25°C, plus préférentiellement de 4°C à 15°C, en particulier 4 °C, pendant un temps déterminé, en fonction dudit organe ou

dudit tissu, ou desdites cellules d'organes ou de tissus, dans une composition définie ci-dessus.

L'expression « temps déterminé, en fonction dudit organe ou dudit tissu, ou desdites cellules d'organes ou de tissus, » désigne un temps de conservation qui est spécifique et dépend de l'organe utilisé.

Le temps acceptable pour assurer la reprise de fonction ultérieure du greffon varie d'un organe à l'autre. Par exemple, il est d'environ 4-5 heures pour le coeur, 4-6 heures pour le poumon, 6 heures pour l'intestin, 10-16 heures pour le foie, 24-35 heures pour le rein et 12-18 heures pour le pancréas. Toutes ces valeurs sont bien connues de l'homme du métier et peuvent être trouvées par exemple dans la thèse de Melle Delphine Fornas (Thèse de Melle Delphine Fornas soutenue le 15 juin 2001 : Solution de préservation d'organe : descriptif, statut réglementaire et enregistrement en Europe; Université Claude Bernard - Lyon I ; Faculté de Pharmacie ; Institut des Sciences Pharmaceutiques et Biologiques).

Il est également décrit un procédé de culture de cellules d'organes ou de tissus d'organes de vertébrés, ou d'invertébrés comprenant une étape de
mise en culture de cellules dans une composition telle que définie ci-dessus.

Selon un mode de réalisation préféré le procédé de conservation d'un organe, défini ci-dessus, comprend les étapes suivantes:

- prélèvement dudit organe;
- rinçage dudit organe ou dudit tissu, ou desdites cellules d'organes ou de tissus, à une température comprise de 4°C à 37°C, préférentiellement de 15°C à 37°C, plus préférentiellement de 25°C à 37°C, avec une composition définie ci-dessus;
- conservation en statique ou en perfusion dynamique dudit organe à une température comprise de 4°C à 37°C, préférentiellement de 15°C à 37°C, plus préférentiellement de 25°C à 37°C, en particulier 37°C, pendant un temps déterminé, en
fonction dudit organe, dans une composition définie ci-dessus.

L'expression « temps déterminé, en fonction dudit organe» désigne un temps de conservation qui est spécifique et dépend de l'organe utilisé, comme indiqué ci-dessus.

Il est également décrit un procédé de culture de
cellules de vertébrés, comprenant les étapes suivantes:

- prélèvement desdites cellules sur un vertébré;
- mise en culture à une température comprise d'environ 4°C à environ 37°C, préférentiellement d'environ 15°C à environ 37°C, plus préférentiellement d'environ 25°C à environ 37°C, en particulier environ 37°C, pendant un temps déterminé ou non, en fonction des cellules, dans une composition définie ci-dessus ;
- récolte des cellules par rinçage et lyse du tapis cellulaire.

L'expression «temps déterminé, en fonction des cellules» désigne un temps de culture qui est spécifique et dépend du type de cellules utilisé, de la lignée cellulaire utilisée.

Par exemple, il est en général compris de quelques heures à environ une semaine pour des cultures de cellules telles que des cellules sanguines comme les polynucléaires neutrophiles.

Ces cellules ne peuvent habituellement pas être maintenues en culture indéfiniment, notamment à cause de leur nombre limité de divisions (limite de Hayflick).

Pour des cultures primaires, culture de cellules qui proviennent directement d'un tissu, il est en général compris de quelques jours à plusieurs semaines.

Cette première culture pourra, par la suite, donner lieu à des cultures dites "secondaires", lors de l'atteinte de la confluence de la culture primaire.

Enfin, dans le cas de cellules immortelles, ce temps n'est pas déterminé et peut théoriquement être infini.

Il est également décrit un procédé de culture de cellules d'invertébrés, comprenant les étapes suivantes:

- prélèvement desdites cellules sur un invertébré;
- mise en culture à une température comprise d'environ 4°C à environ 37°C, préférentiellement d'environ 4°C à 25°C, plus préférentiellement d'environ 4°C à environ 15°C, en particulier environ 4°C, pendant un temps déterminé, en fonction des cellules, dans une composition définie ci-dessus;
- récolte des cellules par rinçage et lyse du tapis cellulaire.

L'expression « temps déterminé, en fonction des cellules» désigne un temps de conservation qui est spécifique et dépend du type de cellules utilisé, de la lignée cellulaire utilisée.

## DESCRIPTION DES FIGURES

La figure 1 représente la cinétique de dissociation de l'hémoglobine de *Arenicola marina* (HbAm) dans quatre milieux de conservation d'organes (carré blanc :UW, cercle noir :IGL1, croix :Celsior, triangle blanc :BMPS Belzer).
Le carré gris correspond à la stabilité de l'hémoglobine de *Arenicola marina* dans un tampon servant à la purification de la molécule.
La figure 2 représente les propriétés fonctionnelles de l'hémoglobine de *Arenicola marina* (HbAm) dans différents milieux de conservation d'organes.
Pour chaque milieu, la mesure de la $P_{50}$ (traits pleins) représentant l'affinité de l'hémoglobine de *Arenicola marina* pour l'oxygène ainsi que la mesure de la $n_{50}$ (traits pointillés) représentant la coopérativité sont représentées.

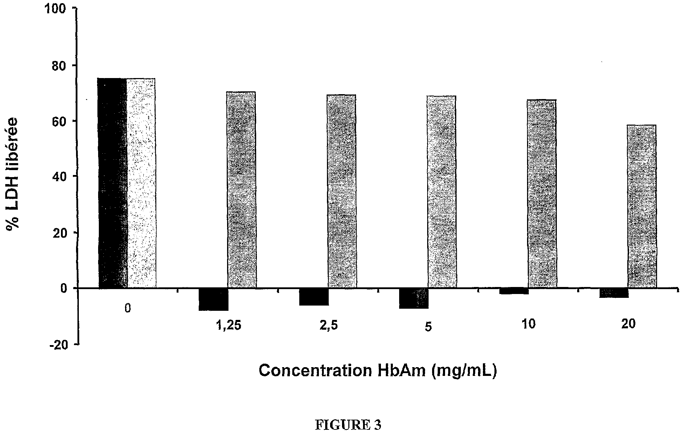

La figure 3 représente le pourcentage de lactate déshydrogénase (LDH) libérée dans le surnageant de conservation par les cellules de tubules rénaux de porc LLC PK1 (axe des ordonnées) après 2h à 4°C en fonction de la concentration en hémoglobine de *Arenicola marina* (1,25 mg/ml à 20 mg/ml) dans un milieu de conservation d'organe (ViaSpan®, Bristol-Myers-Squibb).
Les colonnes en noir représentent les résultats obtenus pour les différentes concentrations d'HbAm, les colonnes en gris représentent les résultats obtenus avec le tampon seul dans le ViaSpan®, en l'absence d'HbAm.

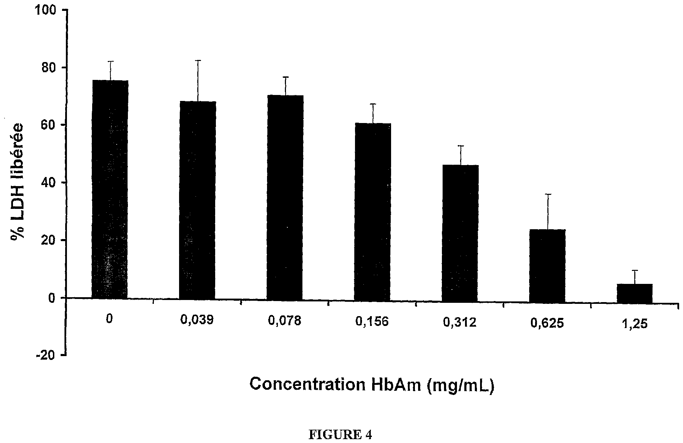

La figure 4 représente le pourcentage de lactate déshydrogénase détecté (LDH) libérée dans le surnageant de conservation par les cellules de tubules rénaux de porc LLC PK1 (axe des ordonnées) après 24 h à 4°C en fonction de la concentration en hémoglobine de *Arenicola marina* (0,039 mg/ml à 1,25 mg/ml ; axe des abscisses) dans un milieu de conservation d'organe (ViaSpan®, Bristol-Myers-Squibb).
Les colonnes en noir représentent les résultats obtenus pour les différentes concentrations d'HbAm.

## PARTIE EXPERIMENTALE

**Exemple 1 : Stabilité de l'hémoglobine de *Arenicola marina* dans différents milieux de conservations d'organes.**

Ces études ont été réalisées au laboratoire afin d'évaluer la stabilité de HbAm dans différents milieu de conservations d'organes (solutions colloïdales couramment utilisées lors de transplantation d'organes et fournies par le laboratoire du Pr. T. Hauet). Ces solutions sont UW®, IGL1®, Celsior®, et BMPS Belzer®.

Les analyses cinétiques ont été réalisées sur 48h à partir de l'aire sous courbe du chromatogramme et du pourcentage à 414nm des différentes sous-unités (correspondant à l'absorbance de l'hème). Le pourcentage de HbAm au cours du temps suit une loi mono-exponentielle :

$$\frac{[HbAm]_t}{[HbAm]_{t0}} = \exp(-k_d t)$$

où $k_d$ est la constante de dissociation de HbAm.

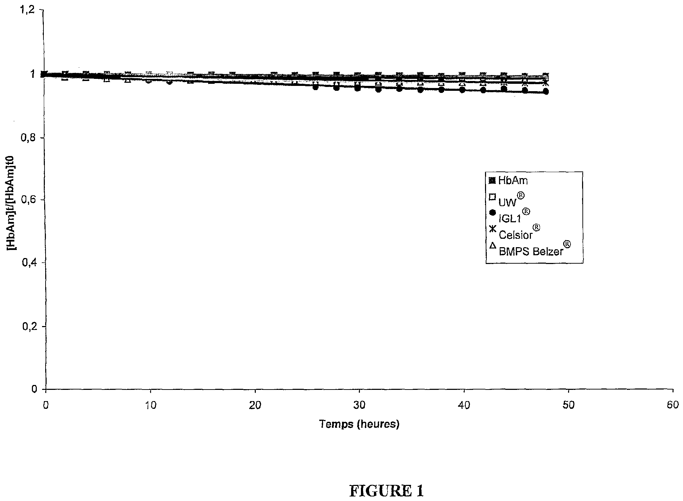

Ces études ont révélé que dans ces différents milieux de conservations d'organes, l'HbAm est stable, non oxydée et fonctionnelle sur une période d'au moins 48 heures, ce qui est totalement compatible avec la durée de vie des organes en attente de transplantation (figure 1).

Les constantes de dissociation et le temps de demi-vie obtenus pour les différentes solutions indiquent que HbAm est stable dans ces différents milieux de conservations d'organes testées sur 48 heures (dissociation < 3%, Tableau I).

Tableau I : Constantes de dissociation et temps de demi-vie de HbAm dans les différents milieux testés.

|  | HbAm | UW® | IGL1® | Celsior® | BMPS Belzer® |
|---|---|---|---|---|---|
| $k_d$ (h$^{-1}$) | 0.0001 | 0.0002 | 0.0011 | 0.0004 | 0.0006 |
| $T_{1/2}$ (h) | ∞ | ∞ | 625 | 1154 | 1705 |

**Exemple 2 : Fonctionnalité de l'hémoglobine de *Arenicola marina* dans différents milieu de conservations d'organes.**

La mesure de la $P_{50}$ a été effectuée selon la technique de l'hemox (A. Toulmond et al., Biol. Bull. 179 366-373 (1990)) à 4° et sur 48h.
La mesure de la $n_{50}$ a été effectuée sur les courbes de saturation par l'oxygène d'un pigment respiratoire, obtenues à partir de la technique de l'hemox.

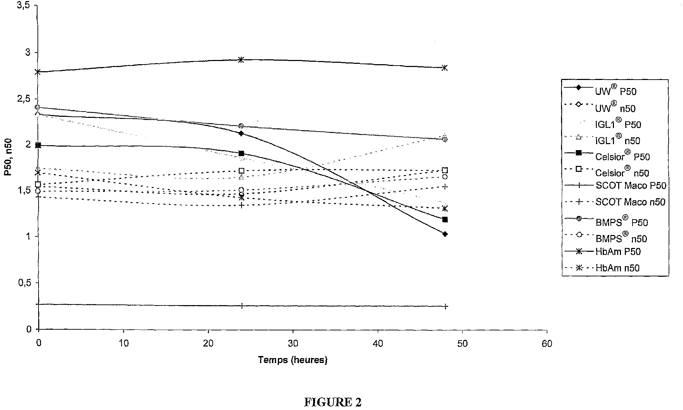

Les résultats obtenus présentés sur la figure 2 montrent que HbAm est fonctionnelle dans les différents milieux de conservation d'organe testés comme l'indiquent les valeurs de P50 et n50 observée (UW, IGL1, Celsior, Scot Maco, BPMS

L'affinité de HbAm pour $O_2$ est forte, comprise entre 1 et 3 et ~0,2 dans SCOT Maco. L'affinité est légèrement plus importante dans les différents milieux de conservation d'organe et particulièrement dans SCOT Maco et augmente légèrement sur 48 heures.

La coopérativité est quant à elle constante ~1,5 entre les différents milieux de conservation ainsi que dans le temps.

**Exemple 3 : Test d'efficacité de l'hémoglobine de *Arenicola marina* à une concentration comprise de 1,25mg/ml à 20 mg/ml, dans un modèle *in vitro* de conservation à froid de cellules rénales**

**1) Conservation à froid de la lignée cellulaire rénale**

**- Matériel biologique**

Les expériences ont été réalisées sur la lignée cellulaire de tubules rénaux de porcs LLC-PK1 (CL-101, Lot 1928865) (ATCC, LGC-Promochem, Molsheim, France) gracieusement fournie par le Laboratoire Inserm E0324 'Ischémie-reperfusion en transplantation rénale' de Poitiers dirigé par le Pr. G. Mauco.

La lignée LLC-PK1 est une lignée de cellules non transformées, établie à partir de cellules épithéliales de tubes contournés proximaux de rein de porc.

- Culture des cellules **LLC-PK1**

Les cellules LLC-PK1 sont cultivées en milieu M199 (Ref 31150, Gibco-BrL, Invitrogen Life Technologie) supplémenté avec 3% de sérum de veau foetal (F7524, Lot 085K3397, Sigma-Aldrich), 100 U/mL de pénicilline et 100 $\mu$g/mL streptomycine (P4333, Sigma-Aldrich) et de 2 mM L-Glutamine (25030, Gibco-BrL). Les cellules sont cultivées à 37°C sous une atmosphère humide contenant 95% d'air et 5% de $CO_2$.

**Conservation à froid des cellules LLC-PK1**

Pour les expériences de conservation à froid, les cellules sont ensemencées en plaque de culture 6 puits (140675, Nunc) à une concentration de $2 \times 10^5$ cellules/mL dans 2 mL de milieu de culture pour chaque puits. Après 48 h de culture, le surnageant est éliminé et deux lavages du tapis cellulaire sont réalisés avec un tampon phosphate salin (PBS, 70011, GIBCO-BrL). Les cellules sont ensuite conservées pendant 24 h à 4°C en présence de 1,2 mL d'une solution de conservation commerciale, la solution UW (ViaSpan®, Bristol-Myers-Squibb), préalablement additionnée d'HbAm à des concentrations de 0, 1.25, 2.5, 5, 10 ou 20 mg/ml.

**2) Détection de la libération de la lactate déshydrogénase (LDH)**

L'impact de l'addition de l'HbAm conservée en Tampon de stockage Hemorgan sur la viabilité des cellules LLC-PK1 conservées pendant 24 h à 4°C dans la solution UW a été étudié. La viabilité cellulaire a été étudiée par détection de la quantité de la LDH présente dans le tapis cellulaire après conservation à froid en comparaison à la quantité de LDH initialement présente dans le tapis avant l'étape de conservation.

En effet, la libération de cette enzyme dans le milieu extracellulaire est le reflet de la perméabilisation de la membrane plasmique des cellules et par conséquent de la mort cellulaire.

- Mode opératoire

Après 24 h de conservation, le surnageant est éliminé, et le tapis de cellules est ensuite rincé 3 fois avec 2 mL de PBS puis les cellules adhérentes sont lysées dans 1,2 mL de PBS contenant 0,1% de Triton®X-100 (X100, Sigma-Aldrich). La suspension obtenue après grattage du tapis cellulaire est soniquée pendant 10 sec avec un appareil à ultrasons puis centrifugée à 1 000 g pendant 7 min.

La quantité de LDH présente dans le tapis cellulaire est déterminée par un dosage colorimétrique selon les instructions du fournisseur (TOX7, Sigma-Aldrich). Ce dosage est basé sur la réduction du NAD par la LDH lors de la transformation du pyruvate en lactate. Brièvement, 25 $\mu$L de l'échantillon à doser sont déposés dans une plaque 96 puits avant l'addition de 25 $\mu$L d'un mélange réactionnel contenant 1 volume de substrat (L2402), 1 volume de cofacteur (L2527) et 1 volume de colorant (L2277). Le mélange est ensuite homogénéisé délicatement et incubé 5 min à température ambiante dans le noir. La réaction est arrêtée par addition de 5 μL de HCl 1N. L'absorbance mesurée par photométrie à 490 nm et 630 nm (référence plastique) (ELx800™, Biotek couplé au logiciel Gen5®) est directement proportionnelle à la quantité d'enzyme présente dans l'échantillon. Chaque échantillon est dosé en doublet et une moyenne de la différence de l'absorbance (DO490-DO630) est effectuée.

- Résultats

Les résultats sont exprimés en pourcentage de la quantité de LDH détectée dans le tapis des cellules conservées à 4°C par rapport à la quantité de LDH détectée dans le tapis avant la période de conservation à froid (cellules à T0). Le pourcentage de libération de LDH est alors calculé selon le rapport :

$$100 - [(LDH\ tapis\ cellulaire) \times 100 / (LDH\ cellules\ à\ T0)]\ (Figure\ 3\ et\ Tableau\ II)$$

| HbAm | UW | 1,25g/L | 2,5g/L | 5g/L | 10g/L | 20g/L |
|---|---|---|---|---|---|---|
| MOYENNE | 74 | -6 | -3 | -3 | -2 | -5 |
| ECART-TYPE | 9 | 6 | 4 | 8 | 4 | 2 |

| Tp Am | UW | 25μL | 50μL | 100μL | 200μL | 300μL |
|---|---|---|---|---|---|---|
| MOYENNE | 74 | 72 | 69 | 72 | 70 | 62 |
| ECART-TYPE | 9 | 5 | 6 | 3 | 5 | 4 |

Tableau II : Pourcentage de libération de LDH obtenu pour les différentes concentrations en HbAm ou en tampon (les résultats correspondent à la moyenne de trois essais).

- Conclusion

La conservation des cellules LLC-PK1 pendant 24 h à 4°C dans la solution UW (Viaspan) induit une mort cellulaire importante (74%±9).
De plus, la présence du Tampon de stockage Hemorgan dans la solution de conservation UW ne modifie pas ou peu la viabilité des cellules rénales.
En revanche, la présence d'HbAm lors de la conservation, et ceci dès une concentration de 1,25 g/L de la molécule, protège totalement la viabilité du tapis cellulaire.

**Exemple 4: Test d'efficacité de l'hémoglobine de *Arenicola marina* à une concentration comprise de 0,039mg/ml à 1,25 mg/ml, dans un modèle *in vitro* de conservation à froid de cellules rénales**

Le matériel biologique, la culture des cellules et la conservation à froid sont préparés et effectués de la même façon que dans l'exemple 3.

**- Concentration en HbAm :**

Les cellules sont conservées pendant 24 h à 4°C en présence de 1,2 mL d'une solution de conservation commerciale, la solution UW (ViaSpan®, Bristol-Myers-Squibb), préalablement additionnée d'HbAm à des concentrations de 0, 0,039, 0,078, 0,156, 0,312, 0,625 ou 1,25 mg/mL.

**- Résultats**

Les résultats sont exprimés en pourcentage de la quantité de LDH détectée dans le tapis des cellules conservées à 4°C par rapport à la quantité de LDH détectée dans le tapis avant la période de conservation à froid (cellules à T0). Le pourcentage de libération de LDH est alors calculé selon le rapport :

$$100 - [(LDH\ tapis\ cellulaire) \times 100 / (LDH\ cellules\ à\ T0)]\ (Figure\ 4\ et\ Tableau\ III)$$

Tableau III : Pourcentage de libération de LDH obtenu pour les différentes concentrations en HbAm (les résultats correspondent à la moyenne de trois essais).

| HbAm | UW cel | 0,039g/LC | 0,078g/L | 0,156g/L | 0,312g/L | 0,625g/L | 1,25g/L |
|---|---|---|---|---|---|---|---|
| MOYENNE | 76 | 69 | 71 | 62 | 47 | 25 | 7 |
| ECART-TYPE | 7 | 14 | 6 | 7 | 7 | 12 | 5 |

### - Conclusion

La conservation des cellules LLC-PK1 pendant 24 h à 4°C dans la solution UW (ViaSpan®, Bristol-Myers-Squibb) induit une mort cellulaire importante (76%±7). La libération de LDH des cellules tubulaires rénales est remarquablement diminuée en présence d'HbAm et ceci de manière dose-dépendante. Ainsi, dès 0,625 mg/ml, HbAm protège les cellules rénales (25%±12 *versus* 76%±7) et à une concentration de 1,25 g/L, HbAm protège totalement les cellules rénales de la mort cellulaire induite par 24 h de conservation à froid (7%±5 *versus* 76%±7).

### Revendications

1. Composition comprenant:

    - au moins une globine et/ou au moins un protomère de globine et/ou au moins une hémoglobine extracellulaire d'animal invertébré choisi parmi le phylum des annélides, à une concentration, par rapport au volume final, comprise de 0,625 mg/ml à 100 mg/ml, et
    - un milieu de conservation d'organe, ledit milieu étant capable de protéger les organes des effets délétères de l'ischémie en satisfaisant les besoins métaboliques minimums des organes et ledit milieu étant une solution aqueuse contenant des électrolytes et des imperméants.

2. Composition selon la revendication 1, **caractérisée en ce que** la globine et/ou au moins le protomère de globine et/ou au moins l'hémoglobine extracellulaire est d'*Arenicola marina.*

3. Composition selon la revendication 1 ou 2, **caractérisée en ce que** ledit milieu de conservation d'organe contient en outre des colloïdes.

4. Composition selon l'une quelconque des revendications 1 à 3, **caractérisée en ce que** ladite concentration par rapport au volume final est de 0,625 mg/ml à 20 mg/ml, particulièrement de 0,625 mg/ml à 5 mg/ml, plus particulièrement de 1,25 mg/ml.

5. Composition selon l'une quelconque des revendications 1 à 4, **caractérisée en ce que** lesdits électrolytes sont choisis parmi le potassium, le sodium, le magnésium, le calcium, le chlorure ou le sulfate.

6. Composition selon l'une quelconque des revendications 1 à 5, **caractérisée en ce que** lesdits imperméants sont choisis parmi le mannitol, le raffinose, le saccharose, le glucose, le fructose, le lactobionate, ou du gluconate.

7. Composition selon l'une quelconque des revendications 3 à 6, **caractérisée en ce que** lesdits colloides sont choisis parmi l'albumine, de l'hydroxyéthyl amidon, du polyéthylène glycol ou du dextran 40.

8. Procédé de conservation d'organe ou de tissu, ou de cellules d'organes ou de tissus comprenant une étape de conservation en statique ou en perfusion dynamique dudit organe dans une composition selon l'une des revendications 1 à 7.

9. Procédé de conservation d'un organe ou de tissu, ou de cellules d'organes ou de tissus selon la revendication 8, comprenant les étapes suivantes:

    • rinçage dudit organe ou dudit tissu préalablement prélevés, ou desdites cellules d'organes ou de tissus préalablement prélevées, à une température comprise de 4°C à 15°C, avec une composition selon l'une des revendications 1 à 7,
• conservation en statique ou en perfusion dynamique dudit organe ou dudit tissu, ou desdites cellules d'organes ou de tissus, à une température comprise de 4°C à 15°C, pendant un temps déterminé, en fonction dudit organe ou dudit tissu, ou desdites cellules d'organes ou de tissus, dans une composition selon l'une des revendications 1 à 7.

10. Procédé de conservation d'un organe selon la revendication 8, comprenant les étapes suivantes:

• rinçage dudit organe préalablement prélevé, à une température comprise de 25°C à 37°C, avec une composition définie dans l'une des revendications 1 à 7;
• conservation en statique ou en perfusion dynamique dudit organe à une température comprise de 25°C à 37°C, pendant un temps déterminé, en fonction dudit organe, dans une composition définie dans l'une des revendications 1 à 7.

## Patentansprüche

1. Zusammensetzung umfassend:

- mindestens ein Globin und/oder mindestens einen Globin-Protomer und/oder mindestens ein extrazelluläres Hämoglobin eines wirbellosen Tieres ausgewählt aus dem Stamm der Ringelwürmer, in einer Konzentration, bezogen auf das Endvolumen, die 0,625 mg/mL bis 100 mg/mL umfasst, und
- ein Medium zur Organkonservierung, wobei das Medium in der Lage ist, Organe vor den schädlichen Effekten von Ischämie zu schützen, und dabei die metabolischen Mindestvoraussetzungen der Organe erfüllt und wobei das Medium eine wässrige Lösung ist, die Elektrolyte und Impermeate enthält.

2. Zusammensetzung gemäß Anspruch 1, **dadurch gekennzeichnet, dass** das Globin und/oder der mindestens eine Globin-Protomer und/oder das mindestens eine extrazelluläre Hämoglobin von *Arenicola marina* ist.

3. Zusammensetzung gemäß Anspruch 1 oder 2, **dadurch gekennzeichnet, dass** das Medium zur Organkonservierung zusätzlich Kolloide beinhaltet.

4. Zusammensetzung gemäß einem der Ansprüche 1 bis 3, **dadurch gekennzeichnet,dass** die Konzentration, bezogen auf das Endvolumen, 0,625 mg/mL bis 20 mg/mL ist, insbesondere 0,625 mg/mL bis 5 mg/mL, ferner insbesondere 1,25 mg/mL.

5. Zusammensetzung gemäß einem der Ansprüche 1 bis 4, **dadurch gekennzeichnet, dass** die Elektrolyten ausgewählt sind aus Kalium, Natrium, Magnesium, Calcium, Chlorid oder Sulfat.

6. Zusammensetzung gemäß einem der Ansprüche 1 bis 5, **dadurch gekennzeichnet dass** die Impermeate ausgewählt sind aus Mannitol, Raffinose, Saccharose, Glukose, Fruktose, Lactobionat oder Glukonat.

7. Zusammensetzung gemäß einem der Ansprüche 3 bis 6, **dadurch gekennzeichnet dass** die Kolloide ausgewählt sind aus Albumin, Hydroxyethylamidon, Polyethylenglycol, oder Dextran 40.

8. Verfahren zur Organ- und Gewebekonservierung, oder zur Konservierung von Organ- oder Gewebezellen, umfassend einen statischen Konservierungsschritt oder einen Schritt mit dynamischer Durchströmung des Organs in einer Zusammensetzung gemäß einem der Ansprüche 1 bis 7.

9. Verfahren zur Konservierung eines Organs oder eines Gewebes oder zur Konservierung von Organ- oder Gewebezellen gemäß Anspruch 8, umfassend die folgenden Schritte:

- Spülung des Organs oder des Gewebes vor der Entnahme oder der Organ- oder Gewebezellen vor der Entnahme bei einer Temperatur umfassend 4°C bis 15°C mit einer Zusammensetzung gemäß einem der Ansprüche 1 bis 7,
- Statische Konservierung oder Konservierung durch dynamische Durchströmung des Organs oder des Gewebes oder der Organ- oder Gewebezellen bei einer Temperatur umfassend 4°C bis 15°C für eine bestimmte Zeit, abhängig von dem Organ oder dem Gewebe oder der Organ- oder Gewebezellen in einer Zusammensetzung gemäß einem der Ansprüche 1 bis 7.

10. Verfahren zur Konservierung eines Organs gemäß Anspruch 8, umfassend die folgenden Schritte:

- Spülung des Organs vor der Entnahme, bei einer Temperatur umfassend 25°C bis 37°C mit einer Zusammensetzung definiert in einem der Ansprüche 1 bis 7,
- Statische Konservierung oder Konservierung durch dynamische Durchströmung des Organs bei einer Temperatur umfassend 25°C bis 37°C für eine bestimmte Zeit abhängig von dem Organ, in einer Zusammensetzung wie in einem der Ansprüche 1 bis 7 definiert.

**Claims**

1. Composition comprising:

   - at least one globin and/or at least one globin protomer and/or at least one extracellular hemoglobin of an invertebrate animal selected from the phylum Annelida, at a concentration, relative to the final volume, of 0.625 mg/ml to 100 mg/ml, and
   - an organ storage medium, said medium being capable of protecting the organs from the deleterious effects of ischemia while satisfying the minimum metabolic needs of the organs, and said medium being an aqueous solution containing electrolytes and impermeants.

2. Composition according to claim 1, **characterized in that** the globin and/or at least the globin protomer and/or at least the extracellular hemoglobin is of *Arenicola marina.*

3. Composition according to claim 1 or 2, **characterized in that** said organ storage medium further contains colloids.

4. Composition according to any one of claims 1 to 3, **characterized in that** said concentration relative to the final volume is of 0.625 mg/ml to 20 mg/ml, particularly of 0.625 mg/ml to 5 mg/ml, more particularly of 1.25 mg/ml.

5. Composition according to any one of claims 1 to 4, **characterized in that** said electrolytes are chosen from potassium, sodium, magnesium, calcium, chlorine or sulfate.

6. Composition according to any one of claims 1 to 5, **characterized in that** said impermeants are chosen from mannitol, raffinose, saccharose, glucose, fructose, lactobionate or gluconate.

7. Composition according to any one of claims 3 to 6, **characterized in that** said colloids are chosen from albumin, hydroxyethyl starch, polyethylene glycol or dextran 40.

8. Process for storage of an organ or tissue, or of organ or tissue cells, comprising a step of static or dynamic perfusion storage of said organ in a composition according to any one of claims 1 to 7.

9. Process for storage of an organ or tissue, or of organ or tissue cells according to claim 8, comprising the following steps:

   - rinsing of said organ or said tissue previously removed, or of said organ or tissue cells previously removed, at a temperature of 4°C to 15°C, with a composition according to any one of claims 1 to 7;
   - static or dynamic perfusion storage of said organ or said tissue, or of said organ or tissue cells, at a temperature of 4°C to 15°C, for a defined time, depending on said organ or tissue, or of said organ or tissue cells, in a composition according to any one of claims 1 to 7.

10. Process for storage of an organ according to claim 8, comprising the following steps:

    - rinsing of said organ previously removed, at a temperature of 25°C to 37°C, with a composition according to any one of claims 1 to 7;
    - static or dynamic perfusion storage of said organ, at a temperature of 25°C to 37°C, for a defined time, depending on said organ, in a composition according to any one of claims 1 to 7.

**FIGURE 1**

**FIGURE 2**

FIGURE 3

FIGURE 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0192320 A
- DE 10220990
- WO 2007085596 A
- US 7220538 B
- US 6994654 B
- US 20060063142 A
- US 6642045 B
- WO 0101774 A

**Littérature non-brevet citée dans la description**

- **N.B. TERWILLIGER.** Molecular Structure of the extracellular heme proteins. Springer-Verlag, 1992, vol. 13, 193-229
- **J.N. LAMY ; B.N. GREEN ; A. TOULMOND ; J.S. WALL ; R.E. WEBER ; S.N. VINOGRADOV.** *Chem. Rev.,* 1996, vol. 96, 3113-3124
- **J. ROCHE ; M. BESSIS ; J.P. THIERY.** *Biochim. Biophys. Acta,* 1960, vol. 41, 182-184
- **J. ROCHE ; M.T. BESSIS.** *C. R. Soc. Biol.,* 1960, vol. 154, 73-80
- **O. LEVIN.** *J. Mol. Biol.,* 1963, vol. 2, 95-101
- **J. ROCHE.** Electron microscope studies on high molecular weight erythrocruorins (invertebrate hemoglobins) and chlorocruorins of annelids. Pergamon Press, 1965, vol. 62-80
- **E.F.J. VAN BRUGGEN ; R.E. WEBER.** *Biochim. Biophys. Acta,* 1974, vol. 359, 210-212
- **O.H. KAPP ; A.V. CREWE.** *Biochim. Biophys. Acta,* 1984, vol. 789, 294-301
- **F. DE HAAS ; F. ZAL ; V. YOU ; F.H. LALLIER ; A. TOULMOND ; J.N. LAMY.** *J. Mol. Biol.,* 1996, vol. 264, 111-120
- **F. DE HAAS ; F. ZAL ; F.H. LALLIER ; A. TOULMOND ; J.N. LAMY.** *Proteins-structure fonction and genetics,* 1996, vol. 3, 241-256
- **F. DE HAAS ; N. BOISSET ; J.C. TAVEAU ; O. LAMBERT ; S.N. VINOGRADOV ; J.N. LAMY.** *Biophys. J.,* 1996, vol. 70, 1973-1984
- **F. ZAL ; B. N. GREEN ; F.H. LALLIER ; S.N. VINOGRADOV ; A. TOULMOND.** *Eur. J. Biochem.,* 1997, vol. 243, 85-92
- **ROUSSELOT et al.** *Biotechnology Journal,* 2006, vol. 1 (3), 333-345
- **BADET.** L'utilisation des liquides de conservation en transplantation rénale. *Prog Urol,* 2006, vol. 16, 25-31
- **BELZER F.O. ; SOUTHARD J.H.** Principles of solid-organ preservation by cold storage. *Transplantation,* 1988, vol. 45 (4), 673-676
- **BELZER.** Southard, Principles of solid-organ preservation by cold storage. *Transplantation 1988,* 1988, vol. 45 (4), 673-676
- **SAVITSKY JP ; DOCZI J ; BLACK J ; ARNOLD JD.** A clinical safety trial of stroma-free hemoglobin. *Clin Pharmacol Ther,* 1978, vol. 23, 73-80
- **CHAN WL ; TANG NL ; YIM CC ; LAI FM ; TAM MS.** New features of renal lesion induced by stroma free haemoglobin. *Toxicol Pathol,* 2000, vol. 28, 635-642
- **HENDRY EB.** Osmolarity of human serum and chemical solution of biological importance. *Clin. Chem.,* 1961, vol. 2, 156-164
- **JENSEN, FB ; WANG T. ; BRAHM, J.** *Acute and chronic influence of temperature on red blood cell anion exchange,* 2001, vol. 204, 39-45
- **GOODNOUGH.** *Clin Orthop RelatRes,* Décembre 1998, 89-100
- **FLOHÉ & ÖTTING ; FLOHÉ L ; OTTING F.** *Methods Enzymol,* 1984, vol. 105, 93-104
- **TOULMOND, A.** Recherches sur la physiologie respiratoire de l'Annélide Polychète. *Arenicola marina,* 1975
- **RINKEVICH B.** *Mar Biotechnol,* Septembre 2005, vol. 7 (5), 429-39
- **A. TOULMOND et al.** *Biol. Bull.,* 1990, vol. 179, 366-373